# EUROPEAN PATENT SPECIFICATION

(11) **EP 1 603 928 B1**
(45) Date of publication and mention of the grant of the patent: **01.08.2007**
(21) Application number: 03722339.3
(22) Date of filing: 10.03.2003
(51) Int. Cl.: C07F 9/6558, C07D 405/06, A23L 1/226

(54) **PYRIDINIUM-BETAIN COMPOUNDS AND THEIR USE**
PYRIDINIUM-BETAIN VERBINDUNGEN UND DEREN VERWENDUNG
COMPOSES DE PYRIDINIUM-BETAINE ET LEUR UTILISATION

(43) Date of publication of application: 14.12.2005
(73) Proprietor: NESTEC S.A., 1800 Vevey (CH)
(72) Inventor: BLANK, Imre, CH-1073 Savigny (CH); GRIGOROV, Martin, CH-1066 Epalinges (CH); HOFMANN, Thomas, 48161 Muenster-Roxel (CH)
(74) Representative: Thomas, Alain
(86) International application number: PCT/EP2003/002594
(87) International publication number: WO 2004/081018

(56) References cited:
- WO-A-88/06850
- US-A- 4 530 799

## Description

The so-called umami taste has recently been accepted as the fifth basic taste quality along with the taste modalities sweet, sour, salty, and bitter. This is mainly due to the identification of G protein-coupled receptors for glutamate such as mGluR4 (N. Chaudari et al., Nat. Neurosci. 2000, 3, 113-119.) or the heteromer T1R1+3 receptor (G. Nelson et al., Nature 2002, 416(6877), 199-202), which was reported to be a broadly tuned receptor stimulated by many L-amino acids, in particular also by glutamate. Monosodium glutamate (MSG) is the best-known compound eliciting umami taste (K. Ikeda, J. Tokyo Chem. Soc. 1909, 30, 820-826). Other compounds with similar sensory characteristics belong to the group of purine-5'-nucleotides, such as inosine-5'-monophosphate (IMP) (A. Kuninaka, In: Symposium on Foods: The Chemistry and Physiology of Flavors; Schultz, H. W.; Day, E. A.; Libbey, L. M., Eds.; AVI Publishing Company: Westport, CT, 1967; pp 515-535). These compounds occur in many savoury foods such as meat, fish, seafood, and mushrooms (S. Yamaguchi, J. Food Sci. 1967, 32, 473-478). An interesting property of umami compounds is their mutual taste synergism. The synergistic effects between MSG and IMP have been investigated and reported in the literature (S. Yamaguchi et al., J. Food Sci. 1971, 36, 1761-1765).

The present invention concerns Pyridinium-Betain compounds and their use as taste modifying compounds (taste modulators). The term 'taste modifying' is defined as the ability to enhance or to reduce the sensory properties of taste compounds.

The invention discloses a series of homologous betaine pyridinium compounds and their use as agents with taste-modifying properties. By taste-modifying properties we understand the ability of the compounds to enhance or to reduce the sensory quality of taste-active compounds. These taste-modifying compounds can be used as such or generated *in-situ* by thermal or enzymatic reactions.

The aim of the present invention is to identify compounds having taste modifying properties, for example to enhance the overall umami sensory quality of samples containing taste-active compounds.

The discovery of the compounds was made through the use of molecular modelling and quantitative-structure activity relationship (QSAR) methods. The series was specifically designed to match closely in sapophoric space known enhancers of umami taste, such as inosine monophosphate (IMP) or guanosine monophosphate (GMP). Here we mean by the term sapophore the minimum set of chemical features needed to be present on a molecule in order this to elicit certain taste, in our case umami taste.

The present invention concerns compounds called Pyridinium-Betain compounds of the general formula **(A)**: 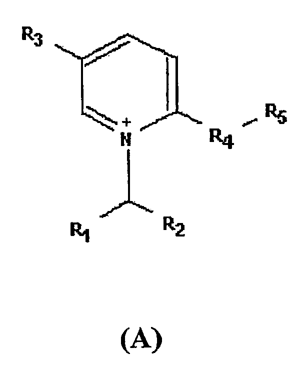 wherein:
**R₁** is H
**R₂** is a chemical group formed by a sugar pentose or hexose ring, substituted at C5 or C6, respectively, with an acid residue taken from the group - phosphoryl, sulfonyl, or carboxyl, and R₂ is connected to the pyridaine ring either at C1 or C2 positions, wherein C1, C2, C5 and C6 belongs to the sugar moiety,
**R₃** is OH, including the ionised form O⁻,
**R₄** is an aliphatic chain (CH₂)ₙ, where n is the chain length in the range from n=0 to n=4,
**R₅** is taken from the group consisting of residues - hydroxy, methoxy, ethoxy, iso-propoxy, propoxy, allyloxy,methyl, ethyl, phenyl, methylthio, ethylthio, ethoxyethylthio, ethoxycarbonylethylthio, furfurylthio, tetrahydrofurfurylthio, isopentenylthio, (beta-methylallyl)thio, (gamma-methylallyl)thio, and derivatives
and wherein the counter-ion is taken from the group consisting of sodium, potassium, ammonium, calcium, magnesium, chloride, nitrate, carbonate, sulphate, phosphate, and the like.

In a preferred embodiment of the compound of the invention, **R₂** is the rest of a sugar phosphate, **R₃** is OH including the ionised form O⁻, **R₄** is CH₂, and **R₅** is a hydroxyl group (OH), including the ionised form O⁻.

The compounds of the general formula **(A)** have zwitterionic character in a broad pH range. As shown below, the zwitterionic structure **(A2)** dominates under slightly acidic and neutral conditions with the negative charge primarily located at the phosphate group attached to the sugar moiety (**R₂** in **(A)**). Under basic conditions represented by the structure **(A3),** the negative charge may be located at the phosphate group attached to the sugar moiety (group **R₂** in **(A))** and at the hydroxyl group directly attached to the pyridinium ring (group **R₃** in **(A)**). Under strongly acidic conditions, both the phosphate and hydroxyl groups are protonated, as shown in the structure **(A1) .** Depending on the pH of an aqueous solution containing the compounds of the general formula **(A),** the structures **(A1), (A2)** and **(A3)** may exist in an equilibrium.

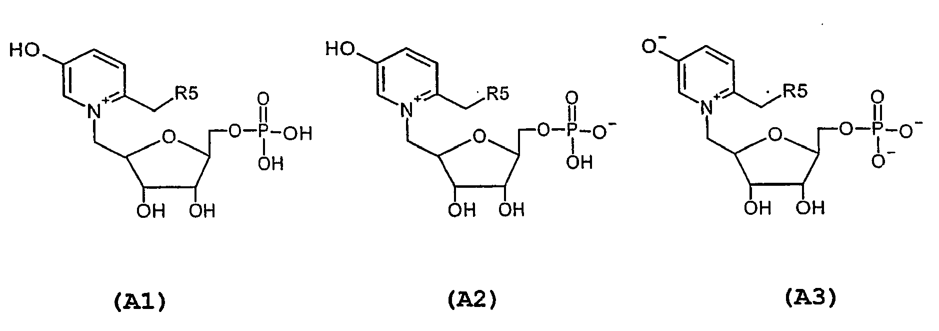

The above-mentioned compounds are reaction products from reducing sugars or their derivatives with amino compounds and their derivatives.

The amount of the compound **(A)** is comprised between 0.01 and 3000 mg/kg of the whole composition.

In addition, the present invention concerns a process for the preparation of the compounds **(A),** wherein said compound is obtained by synthesis using 5-(hydroxymethyl) -2-furanaldehyde (HMF) and the corresponding amino sugar or derivatives thereof. Another way of proceeding is to use HMF producing precursors, such mono- and polysaccharides, and the corresponding amino sugar or derivatives thereof.

The following examples illustrate the invention in more details.

### Rationale

The above-mentioned compounds were designed as umami taste enhancers. This property was deduced by virtual screening of the compounds through a sapophoric model. The model was produced in order to capture most of the information contained in structure-activity data of molecules possessing umami taste enhancing properties. Such molecules were collected through the available literature, such as S. Yamaguchi and K. Ninomyia, Food Rev. Int. 14 (2&3), 123-138, 1989, and references mentioned therein.

### Example 1: Schematic synthesis procedure

Compounds of the general formula **(A)** can be prepared using suitable starting materials and well-known protection and coupling methods described in organic chemistry (J. March, Advanced Organic Chemistry: Reactions, Mechanisms, and Structure, 4th edition, J. Wiley & Sons: New York, 1992) .

As an example, 5-hydroxymethylfurfural (HMF), a well-known sugar degradation product, coupled with an amino sugar derivative results in compound **(A4)** according to the general method described in the literature (Koch et al . , Carbohydrate Chemistry, 1988, 313, 117-123).

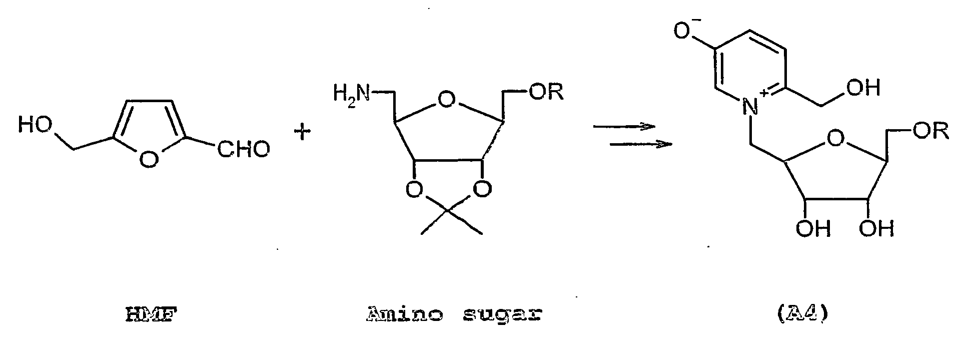

### Example 2: Synthesis via reductive amination of HMF

Alternatively, compounds of the general formula **(A)** can be prepared by reductive amination of 5-hydroxymethylfurfural (HMF) according to the general procedure described in the literature (Müller et al., Tetrahedron, 1998, 54, 10703-10712).

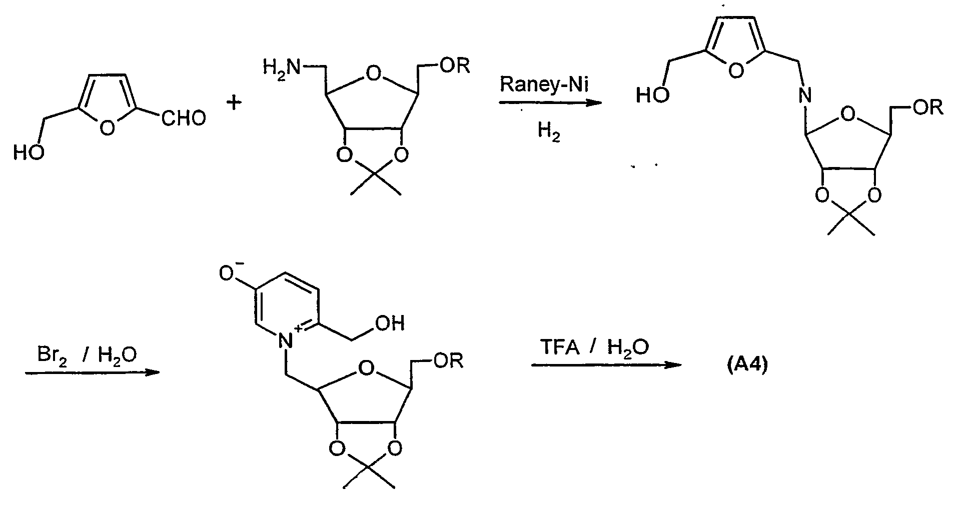

### Example 3: Enhancement of the umami taste modality

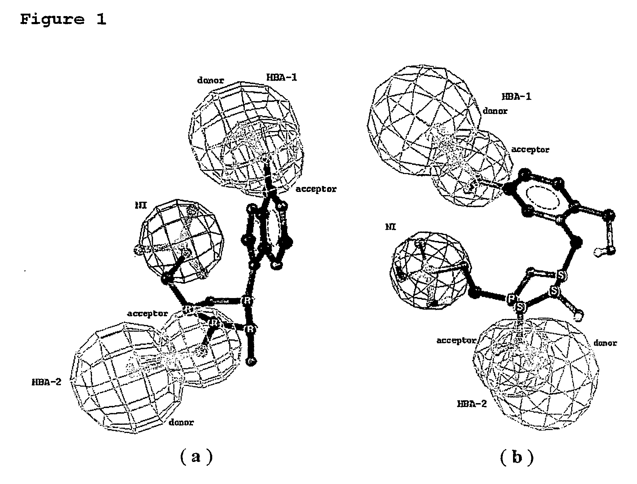

In Figure 1, we show how the well-known umami taste enhancer IMP projects on the optimal sapophore space (a), as well as the mapping in the same space of a proposed betaine-pyridinium compound (b). In light grey are depicted the locations of hydrogen-bond acceptor sites (HBA), while in dark grey are depicted the locations of negatively ionisable groups (NI). The spheres diameters account for tolerances in these positions. Detailed geometric properties of the optimal sapophore are given in Table I.

**Table I**

| | | **Hydrogen Acceptor HBA** | **Bond Site 1, 1** | **Hydrogen Acceptor HBA** | **Bond Site 2, 2** | **Negatively Ionisable Site, NI** |
|---|---|---|---|---|---|---|
| | | **acceptor** | **donor** | **acceptor** | **donor** | |
| **Tolerances,** , | **Å** | 1.60 | 2.20 | 1.60 | 2.20 | 1.60 |
| **Co-ordinates,** | **X** | 4.44 | 6.23 | -1.48 | -1.57 | 2.09 |
| | **Y** | 2.40 | 2.25 | -2.39 | -5.33 | -1.63 |
| | **Z** | -1.30 | 1.10 | 0.28 | 0.90 | -2.22 |

| **Inter-feature distances, Å** | | | | | | |
|---|---|---|---|---|---|---|
| **HBA-1,** | **acceptor** | 0.0 | - | - | - | - |
| | **donor** | 3.0 | 0.0 | - | - | - |
| **HBA-2,** | **acceptor** | 7.8 | 9.0 | 0.0 | - | - |
| | **donor** | 10.0 | 10.9 | 3.0 | 0.0 | - |
| **NI** | | 4.8 | 6.0 | 4.4 | 6.1 | 0.0 |

## Claims

1. Pyridinium-Betain compounds of the following general formula **(A)** 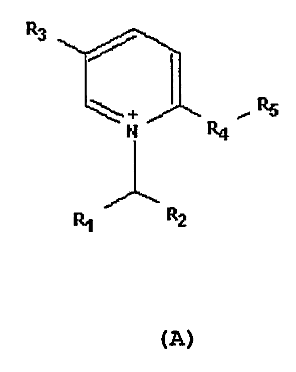 wherein:
**R₁** is H
**R₂** is a chemical group formed by a sugar pentose or hexose ring, substituted at C5 or C6, respectively, with an acid residue taken from the group - phosphoryl, sulfonyl, or carboxyl, and R₂ is connected to the pyridaine ring either at C1 or C2 positions, wherein C1, C2, C5 and C6 belongs to the sugar moiety,
**R₃** is taken from the group consisting of OH, including the ionised form O⁻,
**R₄** is an aliphatic chain (CH₂) ₙ, where n is the chain length in the range from n=0 to n=4,
**R₅** is taken from the group consisting of residues - hydroxy, methoxy, ethoxy, iso-propoxy, propoxy, allyloxy, methyl, ethyl, phenyl, methylthio, ethylthio, ethoxyethylthio, ethoxycarbonylethylthio, furfurylthio, tetrahydrofurfurylthio, isopentenylthio, (beta-methylallyl)thio, (gamma-methylallyl)thio, and derivatives,
and wherein the counter-ion is taken from the group consisting of sodium, potassium, ammonium, calcium, magnesium, chloride, nitrate, carbonate, sulphate, phosphate, and the like.

2. Pyridinium-Betain compounds according to claim 1, wherein **R₂** is the rest of a sugar phosphate, **R₃** is OH including the ionised form O⁻, **R₄** is CH₂, and **R₅** is a hydroxyl group(OH, including the ionised form O⁻ .

3. Pyridinium-Betain compounds according to claim 1, wherein **R₂** is the rest of a sugar phosphate, **R₃** is OH including the ionised form O⁻, **R₄** is CH₂, and **R₅** is furfurylthio radical.

4. The use of a Pyridinium-Betain compound according to any of claims 1 to 3, wherein compound **(A)** is added to a food composition to enhance the umami taste of a compound having said functionality.

5. The use according to claims 4, wherein the food composition is taken from the group consisting of culinary products, and petfood.

6. The use according to any of claims 4, wherein the amount of the compound **(A)** is comprised between 0.01 and 3000 mg/kg of the whole composition.

7. A process for the preparation of the Pyridinium-Betain compounds of claims 1 to 3, wherein compound **(A)** is obtained by synthesis using 5-(hydroxymethyl)-2-furanaldehyde (HMF) and the corresponding amino amino sugars or derivatives thereof.

8. A process for the preparation of the Pyridinium-Betain compounds of claims 1 to 3, wherein compound **(A)** is obtained by reacting HMF producing precursors, such as mono- and polysaccharides, and degradation products thereof with the corresponding amino sugars or derivatives thereof.

## Patentansprüche

1. Pyridinium-Betain-Verbindungen der folgenden allgemeinen Formel **(A)** 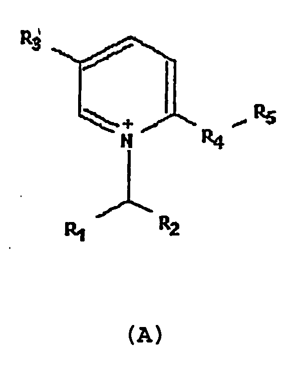 worin:
**R₁** = H ist;
**R₂** eine chemische Gruppe ist, die von einem Zuckerpentose- oder -hexose-Ring gebildet wird, der an C5 bzw. C6 mit einem Säurerest substituiert ist, der aus der Gruppe Phosphoryl, Sulfonyl oder Carboxyl genommen ist, und **R₂** an den Pyridainring entweder über die C1- oder C2-Stellung gebunden ist, wobei C1, C2, C5 und C6 zu der Zuckereinheit gehören,
**R₃** aus der Gruppe genommen ist, die besteht aus OH, einschließlich der ionisierten Form O⁻,
**R₄** eine aliphatische Kette (CH₂)ₙ ist, worin n die Kettenlänge im Bereich von n=0 bis n=4 bedeutet,
**R₅** aus der Gruppe genommen ist, die besteht aus den Resten Hydroxy, Methoxy, Ethoxy, iso-Propoxy, Propoxy, Allyloxy, Methyl, Ethyl, Phenyl, Methylthio, Ethylthio, Ethoxyethylthio, Ethoxycarbonylethylthio, Furfurylthio, Tetrahydrofurfurylthio, Isopentenylthio, (beta-Methylallyl)thio, (gamma-Methylallyl)thio und Derivaten,
und wobei das Gegenion aus der Gruppe genommen ist, die besteht aus Natrium, Kalium, Ammonium, Calcium, Magnesium, Chlorid, Nitrat, Carbonat, Sulfat, Phosphat und dergleichen.

2. Pyridinium-Betain-Verbindungen nach Anspruch 1, wobei **R₂** der Rest eines Zuckerphosphats ist, **R₃** OH, einschließlich der ionisierten Form O⁻ ist, **R₄** CH₂ ist und **R₅** eine Hydroxylgruppe OH, einschließlich der ionisierten Form O⁻, ist.

3. Pyridinium-Betain-Verbindungen nach Anspruch 1, wobei **R₂** der Rest eines Zuckerphosphats ist, **R₃** OH, einschließlich der ionisierten Form O⁻, ist, **R₄** CH₂ ist und **R₅** ein Furfurylthiorest ist.

4. Verwendung einer Pyridinium-Betain-Verbindung nach irgendeinem der Ansprüche 1 bis 3, wobei die Verbindung **(A)** einer Lebensmittelzusammensetzung zugesetzt wird, um den Umami-Geschmack einer Verbindung mit der genannten Funktionalität zu verstärken.

5. Verwendung nach Anspruch 4, wobei die Lebensmittelzusammensetzung aus der Gruppe ausgewählt ist, die besteht aus kulinarischen Produkten und Haustierfutter.

6. Verwendung nach Anspruch 4, wobei die Menge der Verbindung **(A)** im Bereich zwischen 0,01 und 3000 mg/kg der Gesamtzusammensetzung liegt.

7. Verfahren zur Herstellung der Pyridinium-Betain-Verbindungen der Ansprüche 1 bis 3, wobei die Verbindung **(A)** erhalten wird durch eine Synthese unter Verwendung von 5-(Hydroxymethyl)-2-furanaldehyd (HMF) und den entsprechenden Amino-Zuckern oder Derivaten davon.

8. Verfahren zur Herstellung der Pyridinium-Betain-Verbindungen der Ansprüche 1 bis 3, wobei die Verbindung **(A)** erhalten wird durch Umsetzung von HMF-liefernden Vorläufern, wie beispielsweise Mono- und Polysacchariden und Abbauprodukten davon, mit den entsprechenden Aminozuckern oder Derivaten davon.

## Revendications

1. Composés de pyridinium-bétaïne de formule générale **(A)** suivante : 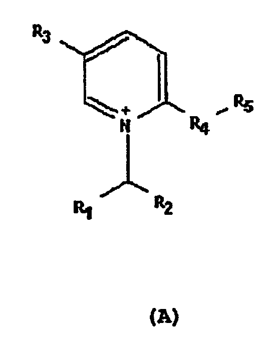 dans laquelle :
**R₁** représente H,
**R₂** représente un groupe chimique formé par un noyau de sucre pentose ou hexose, substitué en position C5 ou
C6, respectivement, avec un résidu acide choisi dans le groupe consistant en des résidus phosphoryle, sulfonyle et carboxyle, et R₂ est connecté au noyau pyridine en position C1 ou C2, les positions C1, C2, C5 et C6 appartenant au groupement sucre,
**R₃** est choisi dans le groupe consistant en un groupe OH, et la forme ionisée O⁻,
**R₄** représente une chaîne aliphatique (CH₂)ₙ dans laquelle n représente la longueur de chaîne allant de n = 0 à n = 4,
**R₅** est choisi dans le groupe consistant en des résidus hydroxy, méthoxy, éthoxy, isopropoxy, propoxy, allyloxy, méthyle, éthyle, phényle, méthylthio, éthylthio, éthoxyéthylthio, éthoxycarbonyléthylthio, furfurylthio, tétrahydrofurfurylthio, isopenténylthio, (bêta-méthylallyl)thio, (gamma-méthylallyl)thio et leurs dérivés,
et dans laquelle l'ion complémentaire est choisi dans le groupe consistant en sodium, potassium, ammonium, calcium, magnésium, chlore, nitrate, carbonate, sulfate, phosphate, et similaires.

2. Composés de pyridinium-bétaïne suivant la revendication 1, dans lesquels **R₂** représente le reste d'un phosphate de sucre, **R₃** représente un groupe OH ou la forme ionisée O⁻, **R₄** représente un groupe CH₂ et **R₅** représente un groupe hydroxyle (OH, ou la forme ionisée O⁻).

3. Composés de pyridinium-bétaïne suivant la revendication 1, dans lesquels **R₂** représente le reste d'un phosphate de sucre, **R₃** représente un groupe OH ou la forme ionisée O⁻, **R₄** représente un groupe CH₂ et **R₅** représente un radical furfurylthio.

4. Utilisation d'un composé de pyridinium-bétaïne suivant l'une quelconque des revendications 1 à 3, dans laquelle le composé **(A)** est ajouté à une composition alimentaire pour augmenter le goût d'umami d'un composé ayant ladite fonctionnalité.

5. Utilisation suivant la revendication 4, dans laquelle la composition alimentaire est choisie dans le groupe consistant en des produits culinaires et des aliments pour animaux de compagnie.

6. Utilisation suivant l'une quelconque des revendications 4, dans laquelle la quantité de composé **(A)** est de 0,01 à 3000 mg/kg de la composition totale.

7. Procédé pour la préparation des composés de pyridinium-bétaïne des revendications 1 à 3, dans lequel le composé (A) est obtenu par synthèse en utilisant du 5-(hydroxyméthyl)-2-furanaldéhyde (HMF) et les sucres aminés correspondants ou leurs dérivés.

8. Procédé pour la préparation des composés de pyridinium-bétaïne des revendications 1 à 3, dans lequel le composé **(A)** est obtenu en faisant réagir des précurseurs produisant HMF, tels que des mono- et polysaccharides, et leurs produits de dégradation, avec les sucres aminés correspondants ou leurs dérivés.
